# EUROPEAN PATENT SPECIFICATION

(11) **EP 1 204 421 B1**
(45) Date of publication and mention of the grant of the patent: **12.11.2008**
(21) Application number: 00954739.9
(22) Date of filing: 18.08.2000
(51) Int. Cl.: A61K 39/00, A61K 39/02, A61K 39/002, A61K 39/385, A61P 31/04, A61P 33/02

(54) **STRESS PROTEIN-PEPTIDE COMPLEXES AS VACCINES AGAINST INTRA-CELLULAR PATHOGENS**
STRESS-PROTEIN-PEPTIDKOMPLEXE ALS IMPFSTOFFE GEGEN INTRAZELLULÄRE PATHOGENE
COMPLEXES D'UN PEPTIDE ET D'UNE PROTEINE DE STRESS EN TANT QUE VACCINS CONTRE LES PATHOGENES INTRACELLULAIRES

(30) Priority: 19.08.1999 GB 9919733
(43) Date of publication of application: 15.05.2002
(73) Proprietor: Immunobiology Limited, Cambridge CB2 4AT (GB)
(72) Inventor: COLACO, Camilo, Anthony, Leo, Selwyn, Cambridge CB2 2JN (GB)
(74) Representative: Ouzman, Beverley Nicola Claire
(86) International application number: PCT/GB2000/003225
(87) International publication number: WO 2001/013943

(56) References cited:
- WO-A-00/10597
- WO-A-98/34641
- HEIKEMA A ET AL: "Generation of heat shock protein-based vaccines by intracellular loading of gp96 with antigenic peptides." IMMUNOLOGY LETTERS, (1997 JUN 1) 57 (1-3) 69-74. , XP000982318
- MULTHOFF G: "HEAT SHOCK PROTEIN 72 (HSP72), A HYPERTHERMIA-INDUCIBLE IMMUNOGENIC DETERMINANT ON LEUKEMIC K562 AND EWING'S SARCOMA CELLS." INTERNATIONAL JOURNAL OF HYPERTHERMIA, vol. 13, no. 1, 1997, pages 39-48, XP000982278 ISSN: 0265-6736

## Description

The present invention relates to a vaccine and a method for producing a vaccine. More specifically, it relates to methods for producing vaccines of shock protein/antigenic peptide fragment complexes from cells infected by intracellular pathogens and the compositions obtained thereby.

### BACKGROUND OF THE INVENTION

An important component of any human immune response is the presentation of antigens to T cells by antigen presenting cells (APCs) such as macrophages, B cells or dendritic cells. Peptide fragments of foreign antigens are presented on the surface of the macrophage in combination with major histocompatibility complex (MHC) molecules, in association with helper molecules, such as CD4 and CD8 molecules. Such antigenic peptide fragments presented in this way are recognised by the T cell receptor of T cells. The interaction of the antigenic peptide fragments with the T cell receptor results in antigen-specific T cell proliferation, and secretion of lymphokines by the T-cells. The nature of the antigenic peptide fragment presented by the APCs is critical in establishing immunity.

Heat shock proteins (HSPs) form a family of highly conserved proteins that are widely distributed throughout the plant and animal kingdoms. On the basis of their molecular weights, HSPs are grouped into six different families: small (hsp 20-30kDa); hsp40; hsp60; hsp70; hsp90; and hsp100. Although HSPs were originally identified in cells subjected to heat stress, they have been found to be associated with many other forms of stress, such as infections, and are thus more commonly known as stress proteins (SPs). For convenience, the initials SP will be used herein to denote in general all forms of stress proteins however produced, and the initials HSP will be used to denote those proteins which have been produced by heat stress.

Members of the mammalian hsp90 family include cytosolic hsp90 (hsp83) and the endoplasmic reticulum counterparts hsp90 (hsp83), hsp87, Grp94 (Erp99) and gp97, see for example Gething et al. (1992) Nature 355:33-45. Members of the hsp70 family include cytosolic hsp70 (p73) and hsp70 (p72), the endoplasmic reticulum counterpart BiP (Grp78), and the mitochondrial counterpart hsp70 (Grp75). Members of the mammalian hsp60 family have only been identified in the mitochondria.

SPs are ubiquitous within cells. One of the roles of SPs is to chaperone peptides from one cellular compartment to another and to present peptides to the MHC molecules for cell surface presentation to the immune system. In the case of diseased cells, SPs also chaperone viral or tumour-associated peptides to the cell-surface, see Li and Sirivastave (1994) Behring Inst. Mitt, 94: 37-47 and Suzue et al. (1997) Proc.Natl.Acad.Sci. USA 94: 13146-51. The chaperone function is accomplished through the formation of complexes between SPs and the antigenic peptide fragments and between SPs and viral or tumour-associated peptide fragments in an ATP-dependent reaction. SPs bind or complex with a wide spectrum of peptide fragments in an ATP dependent manner. The peptides in such complexes appear to be a random mix of peptide fragments. The mixtures and exact natures of the peptide fragments have not been determined. The complex formation of SPs with various peptide fragments has been observed in normal tissues as well and is not a tumour-specific phenomenon, see Srivastava (1994) Experimentia 50: 1054-60.

In a therapeutic context, it has been proposed to use mammalian HSPs as vaccines. WO 97/10000 and WO 97/10001 disclose that a mixture of HSPs isolated from cancer cells or virally infected cells are capable of eliciting protective immunity or cytotoxic T lymphocytes to the cognate tumour or viral antigen. However, in contrast, HSPs isolated from normal cells are unable to elicit such immunity. It is now thought that HSPs are not immunogenic *per se,* but are able to elicit immunity because of their association with tumour or virus specific antigenic peptide fragments that are generated during antigen processing. Specifically, the peptide fragments associated with the HSPs are immunogenic, and are presented to the T cells. HSPs stripped of associated peptide fragments lose their immunogenicity, see Udono, H. and Srivastava, P. K., Journal of Experimental Medicine, 178, page 1391 ff, 1993. To date, the nature of these peptide fragments has not been determined.

It is currently believed that the immunogenicity of SPs results not from the SP per se, but from the complex of peptide fragments associated with the SP. This conclusion is based on a number of characteristics of the complexes. There are no differences in the structure of SPs derived from normal and tumour cells. Certain complexes of the SPs with peptide fragments lose their immunogenicity upon treatment with ATP, Udono et al. (1993) J. Exp. Med. 178: 1391-96. Such loss of immunogenicity is due to dissociation of the complex into its SP and peptide components. The immunogenicity of SP preparations depends upon the presence of phagocytic cells, such as macrophages and other APCs. It is now thought that SPs are taken up by macrophages, and those peptide fragments associated with the SPs are then presented by MHC class I molecules of the macrophage. In this way, a T cell response is initiated.

The use of mammalian HSP/antigenic peptide fragment complexes as vaccines against intracellular pathogens has been disclosed in WO 95/24923. HSPs isolated from viral infected cells have been suggested as a source of antigenic peptides, which could then be presented to T cells. This necessitates the production and purification of HSPs from such cells. The stimulation of cells by heat shock produces a general increase in the level of heat shock proteins.

WO98/34641 discloses treatment of infectious diseases by injection of complexes of heat-shock protein(s) non-covalently bound to antigenic molecules.

In Heikema et al. (1997) cells were infected with recombinant Semliki Forest virus expressing the influenza virus nucleoprotein. Heat-shock protein complexes isolated from these cells have been used for immunization.

Neither in WO98/34641 nor in Heikema et al. (1997) the complexes were obtained after heat or TNF treatment.

However, it has not been suggested that cells may be treated by heat shock or other stresses, to increase intra-cellular levels of the HSPs. This is probably because while it would be desirable to stimulate the production of only a subset of HSPs, which are especially immunogenic, at present there is no way to specifically stimulate cells to produce such a subset of HSPs with enhanced immunogenicity.

### SUMMARY OF THE INVENTION

Therefore, in a first aspect, the present invention provides an in vitro method for producing a vaccine composition comprising an immunogenic determinant active component, characterised in that it comprises the steps of:
a) subjecting cells infected with an intra-cellular bacterial, protozoan or parasitic pathogen to stress with heat or tumour necrosis factor; and
b) extracting SP/antigenic peptide fragment complexes from the stressed cells; and
c) using the extracted products as the immunogenic determinant in the preparation of the vaccine composition.

It is surprising that the treatment of cells infected with an intra-cellular pathogen with heat or tumour necrosis factor produces SPs which are more immunogenic than SPs derived from non-induced cells or cells which have been stressed by other stimuli. A notable aspect of immunity elicited by these induced SPs is the long-term memory compared to that induced by immunisation by other SP subsets. The best memory responses for bacterial pathogens are seen with heat-induced stress proteins and for protozoan and parasitic pathogens with tumour necrosis factor.

The term vaccine is used herein to denote to any composition containing an immunogenic determinant which stimulates the immune system such that it can better respond to subsequent infections. It will be appreciated that a vaccine usually contains an immunogenic determinant and an adjuvant, which non-specifically enhances the response to that determinant.

Preferably, the immunogenic determinant for the present invention is delivered in combination with an adjuvant. Suitable adjuvants are readily apparent to the person skilled in the art, such as Freund's complete adjuvant, Freund's incomplete adjuvant, Quil A, Detox, ISCOMs or squalene. However, it will be appreciated that the vaccine of the present invention may also be effective without an adjuvant. Such a vaccine may be given by any suitable means, such as orally, or by injection.

The terms stress proteins and heat shock protein, as used herein, include those proteins that comprise the GroEL, GroES and DnaK and DnaJ families of bacterial HSPs and related families in other extra-cellular pathogens. These families are named on the basis of the size of the peptides which they encode. The families are highly conserved between species. In addition, many bacteria also express homologues of eucaryotic proteins. Preferably the vaccine contains a plurality of SP/antigenic peptide fragment complexes derived from the stressed pathogen. We particularly prefer that the GroEL, GroES, DnaK and DnaJ families of proteins are used as immunogenic determinants in the present invention, with DnaJ and GroEL most preferred. Preferably the SP complexes have greater than 25% homology and/or 20% identity at the amino acid level to the heat-induced HSP protein families.

The present invention requires that the stress treatment that is used is able to stimulate the presence of SPs within the infected cells. We prefer that for cells infected by protozoan and parasitic pathogens the stress induction is by tumour necrosis factor and particularly tumour necrosis factor-α (TNF-α). For cells infected by bacterial pathogens, we prefer that the stress induction is by heat treatment of the infected cells at a temperature 5-10°C above the normal growth temperature of the uninfected cell. Without being constrained by theory, it is thought that the treatment of the cells infected by intracellular pathogen operates either to induce specifically those HSPs most able to interact with antigenic peptides from the pathogen, or to induce those HSPs which are most easily phagocytosed by APCs, or both.

The optimum conditions for inducing the SPs can readily be determined by simple trial and error and the effect of a change of stimuli assessed using conventional techniques, such as in vivo testing on animals or by other techniques, for example those described in 'Current Protocols in Immunology', Wiley Interscience, 1997.

Where the cells are stressed by treatment with TNF, the TNF is suitably used at 0.5-1000 international units (i.u)/ml of media, preferably about 1-500 i.u/ml. Specifically, for TNF-α we prefer that cells are treated with 10-500 i.u./ml and are then cultured for 10-16 hours. Alternatively, cells may be grown on cytokine-producing feeder-monolayers or induced to produce endogenous TNF. Moreover, the incubation time of cells with the stress stimulus is also variable. We prefer that a 10-16 hour exposure time is used, but this time may be reduced to 2-4 hours in some cases and still be effective.

The means to test for optimum heat or TNF levels and incubation period are readily available to the person skilled in the art. However, it will be appreciated that the exact treatment is not crucial to the invention, as long as the treatment stimulates the production of the desired immunogenic products, notably the SP/antigenic peptide fragment complexes, within the treated cells. Similarly, the other conditions of treatment, such as the length of exposure and cell incubation media are not essential features of the present invention and may be varied depending upon the exact nature of the cell population that is used. Means to vary and optimise these parameters will be readily apparent to the person skilled in the art.

Preferably, the TNF is isolated from the same organism as the cell which is to be treated. Treatment of cells with TNF from the same organism provides optimum stimulation of the sythesis of the SP complexes. However, the use of TNF from other species or individuals may also be useful in up-regulation of the SP levels in cells, to provide suitable SP complexes for use in the present invention.

Any suitable pathogen-infected cell or cell line can be used in the present invention, to provide a source of SP complexes. The infected cells are obtained by infection of an appropriate cell line with the desired pathogen in vitro or by the isolation of cells infected by the pathogen *in vivo*. Cells infected in this way can then be subjected to suitable stress in vitro to produce immunogenic SP complexes suitable for vaccination against that pathogen. This includes recombinant cells that express heterologous antigens from the desired intracellular pathogen. These also include all types of transfected recombinant cells used to produce recombinant vaccines, such as mammalian cell lines transfected with recombinant vectors by standard methods in the art such as electroporation, liposome fusion and calcium phosphate. Furthermore, the invention also includes the formation of the desired SP complexes from eucaryotic cells which express heterologous intracellular pathogen antigens that respond to treatment by heat or TNF. While the antigenic fragments are predominantly proteins and peptides, they can also include carbohydrate, nucleic acid and lipid moieties that bind SPs.

It will be appreciated that the cells to be infected by intra-cellular pathogens for present use can have been modified to enable them to constitutively synthesise the SPs normally induced by the appropriate extra-cellular stress stimuli, namely heat or TNF treatment, by modification of their genetic structure using any suitable recombinant DNA technique, for example those described in 'Current Protocols in Molecular Biology', Wiley Interscience, 1997.

The extraction and purification of protein materials induced from the cellular material by the applied stress, notably the SP/antigenic peptide fragment complexes, from the remaining cellular material can be achieved using any suitable technique. For example, the stress treated material can be disrupted by homogenisation or ultrasonic fragmentation, followed by centrifugation to obtain a crude SP preparation in the supernatant. The crude endogenous SP preparations may be used directly as the vaccine of the invention. Optionally, the SP preparations may be purified further by the use of ADP binding columns or other suitable methods readily available to the person skilled in the art, see for example those described in WO 97/10000 and WO 97/10001.

It will be appreciated that specific immunogenic SP/antigenic peptide fragment complexes can be isolated from the mixture of complexes produced from the stressing of the cellular material to produce a vaccine with is pathogen specific. However, this will usually not be required and the mixture of complexes can be used to induce broad spectrum immunisation. If desired, the specific antigenic peptide fragments can be recovered from the complex, for example by treatment with ATP using conventional techniques.

The SP/antigenic peptide fragment complex of the vaccine of the present invention may be delivered in combination with an adjuvant and in an aqueous carrier. Suitable adjuvants are readily apparent to the person skilled in the art, such as Freund's complete adjuvant, Freund's incomplete adjuvant, Quil A, Detox, ISCOMs or squalene. However, the vaccine compositions of the present invention may also be effective without an adjuvant.

The invention also provides a use of a vaccine of the invention optionally in combination with an adjuvant, in the preparation of a medicament for eliciting an immune response in an animal. The animal is typically a human. However, the invention can also be applied to other mammals such as horses, cattle, goats, sheep or swine, and to birds, notably poultry such as chicken or turkeys.

The vaccine compositions of the present invention may be administered by any suitable means, such as orally, by inhalation, transdermally or by injection and in any suitable carrier medium. However, it is preferred to administer the vaccine as an aqueous composition by injection using any suitable needle or needle-less technique.

The vaccines of the invention may contain any suitable concentration of the SP/antigenic peptide fragment complex. We prefer that the SP complex is administered in the range of 10-600 µg, preferably 10-100 µg, most preferably 25 µg, per Kg of body weight of the animal being treated. It will be appreciated that the vaccine of the invention may be applied as an initial treatment followed by one or more subsequent treatments at the same or a different dosage rate at an interval of from 1 to 26 weeks between each treatment to provide prolonged immunisation against the pathogen.

The following examples are provided to illustrate but not limit the invention. Figures 1 to 3 are Capillary Zone Electrophoretic (CZE) profiles of the SP complexes obtained by various stress methods. Fig 1 is the CZE profile of peptides/polypeptides isolated from constitutive SPs isolated from *M. Tuberculosis* infected mouse peritoneal macrophages; Fig 2 is the CZE profile of peptides/polypeptides isolated from heat-induced SPs isolated from *M. Tuberculosis* infected mouse peritoneal macrophages; and Fig 3 is the CZE profile of peptides/polypeptides isolated from TNF-induced SPs isolated from *M. Tuberculosis* infected mouse peritoneal macrophages.

### Example 1: Preparation of heat-induced SPs:

Cells infected with *M. Bovis* were washed in a serum-free media, such as RPMI (Sigma), then heat-shocked at 45°C for 0.5hr or at 42°C for 5hr and cultured overnight. The cells are then washed in serum-free media, followed by a wash in phosphate buffered saline (PBS). The cells are then re-suspended in homogenisation buffer. The homogenisation buffer may be a hypotonic buffer, such as 10 mM phosphate pH 7.4 with 2mM MgCl₂, after which the cells are then disrupted using a cell homogeniser (e.g. a Dounce or Potter homogeniser, Ultraturrax or Waring blender). Alternatively, the homogenisation buffer may contain detergent, such as PBS with 0.5% Tween, the detergent concentration being between 0.1-1% and suitable to solubilise the cell membrane. The cell lysate is then treated by centrifugation, typically 3-5000g for 5 minutes, to remove the nuclei and cell debris, followed by a high speed centrifugation step, typically 100,000g for 15-30 minutes. The supernatant thus obtained is processed to give an SP/antigenic peptide fragment complex suitable for use in a vaccine. This can be done simply by ammonium sulphate precipitation which uses a 20-70% ammonium sulphate cut. Specifically, 20% (w/w) ammonium sulphate is added at 4°C, the precipitate is discarded, followed by the addition of more ammonium sulphate to bring the concentration to 70% w/w. The protein precipitate is harvested by centrifugation and then dialysed into an appropriate physiological, injectable buffer, such as saline, to remove the ammonium sulphate before use. It will be appreciated that the SP complexes isolated in this way are not purified to homogeneity, but are nevertheless suitable for use as a vaccine component.

If a more purified SP complex preparation is required, the complexes may be purified from the supernatant by affinity chromatography on matrices carrying adenosine diphosphate, such as ADP-agarose or ADP-sepharose. These methods are described in WO 97/10000, WO 97/10001 and WO 97/10002.

The SP complexes may be used at any suitable concentration to provide the immunogenic determinant in the vaccine composition. We prefer that the amount of induced SP complex that is administered is in the range of 10-600 µg, preferably 10-100 µg, most preferably 25 µg per kg of animal body weight.

In order to determine the immunogenicity of the SP complexes, T cell proliferation assays may be used. Suitable assays include the mixed-lymphocyte reaction (MLR), assayed by tritiated thymidine uptake, and cytotoxicity assays to determine the release of ⁵¹Cr from target cells, see 'Current Protocols in Immunology', Wiley Interscience, 1997. Alternatively, antibody production may be examined, using standard immunoassays or plaque-lysis assays, or assessed by intrauterine protection of a foetus, see 'Current Protocols in Immunology'.

### Example 2: Preparation of TNF-induced SPs:

Cell lines infected with the malarial pathogen plasmodium were incubated in a serum-free media, such as RPMI (Sigma), and incubated with TNF-α overnight. Typically, rat liver hepatocytes prepared by collagenase treatment of rat liver tissue, were infected with Plasmodium Berghei by incubation of rat liver cells with 3 times the number of parasite cells, for 5hrs at 35°C. Cells were then overnight at 37°C with or without TNF-α. TNF-induced and control cells were then washed in serum-free media followed by a wash in phosphate buffered saline (PBS).

The cells are then re-suspended in homogenisation buffer and disrupted using a cell homogeniser, by cycles of freeze-thaw or by detergent lysis. The cell lysate is then treated by centrifugation, typically 3-5000g for 5 minutes, to remove the nuclei and cell debris, followed by a high speed centrifugation step, typically 100,000g for 15-30 minutes. The supernatant thus obtained is processed to give an SP complex suitable for use in a vaccine as described in Example 1.

### Example 3: Immunisation with induced SPs; immunity in vaccine recipient:

SPs were prepared as described above and mice and rabbits were vaccinated with 1-10 micrograms of the stress-protein containing extract in phosphate buffered saline and boosted with identical vaccine dosages a month after the primary injection. Induction of immunity to pathogen was assayed by Western blot analysis using total plasmodium or *M. bovis* proteins. Antibody titres of 1:1-10,000 were routinely obtained and cytotoxic T-cell activity directed against pathogen infected cells could also be detected in the immunised mice. Challenge of the rabbits with fixed plasmodium or *M. bovis* at 6, 12 and 18 months periods after the initial immunisations resulted in the production of good antibody responses with titres of 1:1-10 000 indicating good memory responses in the immunised animals.

### Example 4: Comparison of associated peptides in constitutive and induced SP complexes and their use as vaccines

Mouse peritoneal macrophages isolated by peritoneal cavity lavage were infected with *M. tuberculosis* (3 x 10⁶ cells incubated with 10⁷ bacterial cells for 6hrs at 35°C). Infected cell cultures were grown overnight in the presence or absence of lug/ml TNF-α at 37°C, for the isolation of constitutive or TNF-induced SPs, or heat-shocked by incubation at 42°C for 2hrs for the isolation of heat-induced SPs (HSPs). Treated cells were pelleted by centrifugation at 3000g for 5 minutes and re-suspended in lysis solution of 1% Tween in 100mM Tris-HCl, pH8. The cell lysate was centrifuged at 5000g for 5 minutes to remove the nuclei and cell debris, followed by a high speed centrifugation step at 100,000g for 15-30 minutes. SPs and HSPs were prepared from the cleared lysates by ammonium sulphate precipitation as described in Example 1 above.

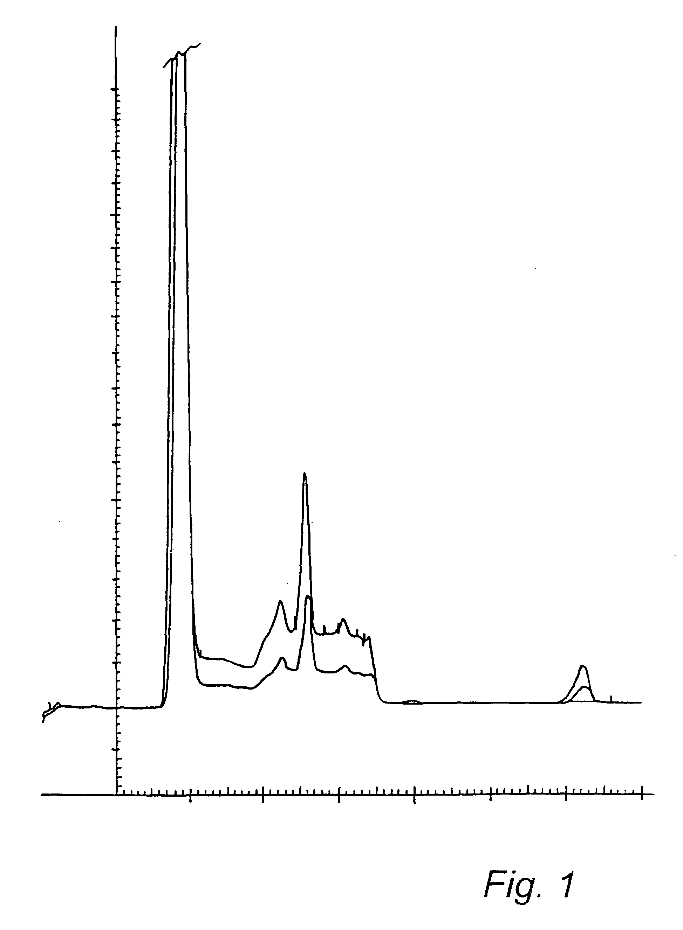

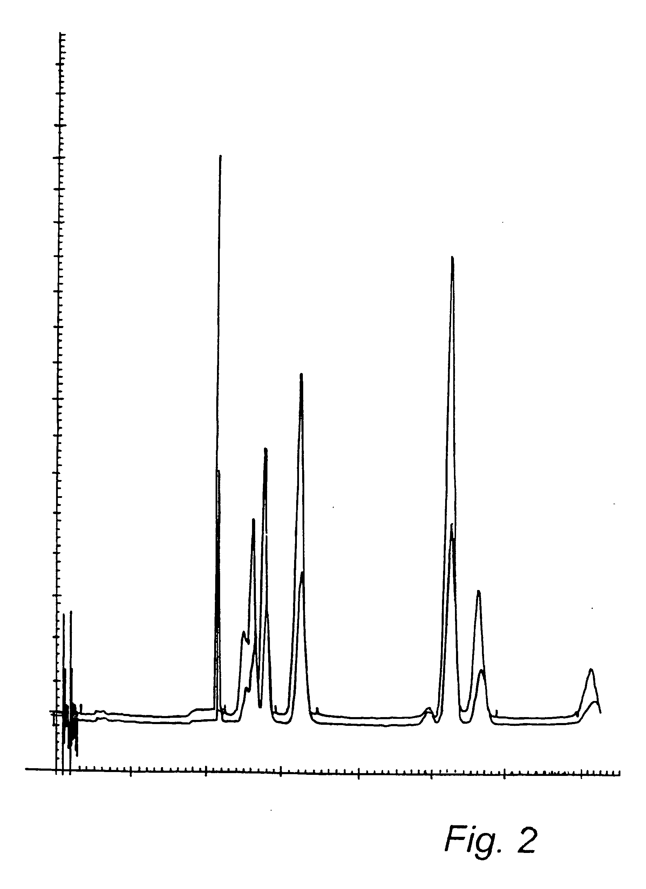

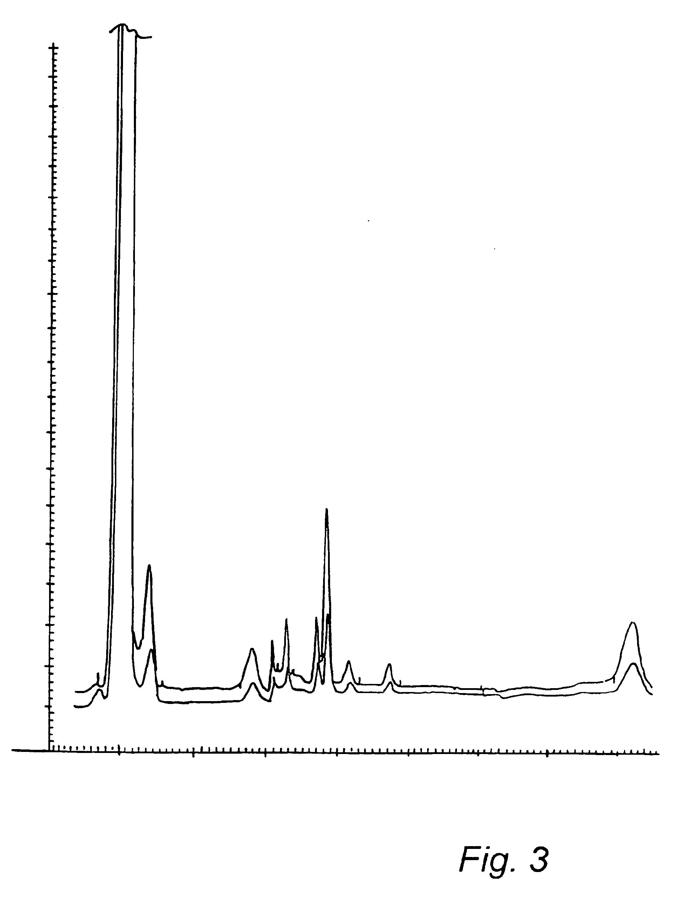

Associated peptides were eluted from the purified HSPs and SPs by re-suspending the precipitated complexes in 10% acetic acid and boiling for 15 minutes to dissociate the complexes. The denatured HSPs and SPs were pelleted in a Beckman airfuge for 30mins in a cold room and the peptide containing supernatants harvested by freeze-drying and analysed by capillary zone electrophoresis using a Beckman CZE system. The CZE profiles of the peptides eluted from the constitutive and the TNF-induced *M. Tuberculosis* SPs and the HSPs were significantly different from each other as shown in Figs 1-3, indicating that all three types of SPs carried distinct families of associated peptides. Immunisation of rabbits with all three types of SPs showed similar antibody titres in animals immunised with the constitutive and TNF-induced SPs compared to significantly higher antibody titres (10-50x) in animals immunised with heat-induced bacterial SPs. Immunisation with admixtures of the eluted peptides and the denatured SPs or HSPs from which they were isolated gave poor antibody responses indicating that the immunity induced required native, intact SP-associated peptide complexes.

### Example 5: Comparison of constitutive and induced SPs as vaccines:

Rat liver hepatocytes were prepared by forcing collagenase digested PVG rat livers through a fine mesh sieve and washing the isolated cells by centrifugation through DMEM tissue culture media. Washed cells were re-suspended at a cell density of 7x10⁶ cells/ml and infected with Plasmodium Berghei by co-culture at 37°C for 4hrs. Infected cells were used to prepare lysates for antibody titre assay, or cultured overnight in the presence or absence of lug/ml TNF-α at 37°C for the isolation of constitutive or TNF-induced SPs. Cells were pelleted by centrifugation at 3000g for 5 minutes and re-suspended in lysis solution of 1% Tween in 100mM Tris-HCl, pH8. The cell lysate was centrifuged at 5000g for 5 minutes to remove the nuclei and cell debris, followed by a high speed centrifugation step at 100,000g for 15-30 minutes. Cleared lysate was then used for antibody titre assays or to isolate SPs for immunisation. Constitutive and TNF-induced SPs were prepared from the cleared lysates by ammonium sulphate precipitation as described in Example 1 above.

Rabbits were immunised with the SPs isolated from constitutive or TNF-induced and heat-induced bacteria re-suspended in phosphate buffered saline without any added adjuvant in either the primary or booster vaccinations. Antibody titres in the immunised animals were assayed by 10-fold serial dilutions using a dot-blot assay on total cell lysates prepared from freshly infected hepatocytes as described analysis above. Animals vaccinated with TNF-induced SPs showed a 10 to 100 fold higher antibody titre than those immunised with constitutive SPs.

## Claims

1. An *in vitro* method for producing a vaccine containing an immunogenic determinant, **characterised in that** it comprises the steps of:
a) subjecting cells infected with an intra-cellular bacterial, protozoan or parasitic pathogen to stress with heat or tumour necrosis factor; and
b) extracting endogenous shock protein/antigenic peptide fragment complexes from the stressed cells; and
c) using the extracted products as the immunogenic determinant in the preparation of the vaccine composition.

2. A method as claimed in claim 1, **characterised in that** the active ingredient of the immunogenic determinant consists predominantly of one or more shock protein/antigenic peptide fragment complexes.

3. A method as claimed in either of claims 1 or 2, **characterised in that** the cells are infected by bacterial pathogens and the stress applied is heat.

4. A method as claimed in claim 3, **characterised in that** the heat stress is achieved by heating to from 5 to 8° above the normal temperature of cultivation of the cells.

5. A method as claimed in claim 1, **characterised in that** the cells are infected by parasitic pathogens and the stress is induced by tumour necrosis factor.

6. A method as claimed in any one of the preceding claims, **characterised in that** the cells have been modified to induce synthesis of stress proteins.

7. A vaccine composition containing an immunogenic determinant, **characterised in that** the immunogenic determinant is produced by a method as claimed in any one of claims 1 to 6.

8. A vaccine composition as claimed in claim 7, **characterised in that** the composition also contains an adjuvant for the immunogenic determinant.

9. A vaccine composition as claimed in claims 7 or 8, **characterised in that** the composition is an aqueous composition.

10. Use of a vaccine composition as claimed in any one of claims 7 to 9 in the preparation of a medicament for eliciting an immune response in an animal.

11. The use as claimed in claim 10, **characterised in that** the vaccine composition is for administration by injection.

## Patentansprüche

1. Ein *In*-*vitro*-Verfahren zur Herstellung eines Impfstoffs, der eine immunogene Determinante enthält, **dadurch gekennzeichnet, dass** es die folgenden Schritte beinhaltet:
a) Aussetzen von Zellen, die mit einem intrazellulären bakteriellen, Protozoen betreffenden oder parasitären Krankheitserreger infiziert sind, zur Belastung mit Wärme oder einem Tumor-Nekrose-Faktor; und
b) Extrahieren der endogenen Schockprotein/Antigenpeptidfragmentkomplexe aus den belasteten Zellen; und
c) Verwenden der extrahierten Produkte als die immunogene Determinante bei der Zubereitung der Impfstoffzusammensetzung.

2. Verfahren gemäß Anspruch 1, **dadurch gekennzeichnet, dass** der Wirkstoff der immunogenen Determinante hauptsächlich aus einem oder mehreren Schockprotein/Antigenpeptidfragmentkomplexen besteht.

3. Verfahren gemäß einem der Ansprüche 1 oder 2, **dadurch gekennzeichnet, dass** die Zellen mit bakteriellen Krankheitserregern infiziert werden und die angewendete Belastung Wärme ist.

4. Verfahren gemäß Anspruch 3, **dadurch gekennzeichnet, dass** die Wärmebelastung durch das Erwärmen auf von 5 bis 8° über der Normaltemperatur zur Kultivierung der Zellen erzielt wird.

5. Verfahren gemäß Anspruch 1, **dadurch gekennzeichnet, dass** die Zellen mit parasitären Krankheitserregern infiziert werden, und die Belastung durch einen Tumor-Nekrose-Faktor induziert wird.

6. Verfahren gemäß einem der vorhergehenden Ansprüche, **dadurch gekennzeichnet, dass** die Zellen modifiziert wurden, um die Synthese von Belastungsproteinen zu induzieren.

7. Eine Impfstoffzusammensetzung, die eine immunogene Determinante enthält, **dadurch gekennzeichnet, dass** die immunogene Determinante durch ein Verfahren gemäß einem der Ansprüche 1 bis 6 hergestellt wird.

8. lmpfstoffzusammensetzung gemäß Anspruch 7, **dadurch gekennzeichnet, dass** die Zusammensetzung auch ein Adjuvans für die immunogene Determinante enthält.

9. Impfstoffzusammensetzung gemäß den Ansprüchen 7 oder 8, **dadurch gekennzeichnet, dass** die Zusammensetzung eine wässrige Zusammensetzung ist.

10. Die Verwendung einer Impfstoffzusammensetzung gemäß einem der Ansprüche 7 bis 9 bei der Zubereitung eines Medikaments zum Auslösen einer Immunreaktion in einem Tier.

11. Verwendung gemäß Anspruch 10, **dadurch gekennzeichnet, dass** die Impfstoffzusammensetzung zur Verabreichung durch Injektion ist.

## Revendications

1. Une méthode in vitro destinée à produire un vaccin contenant un déterminant immunogène, **caractérisée en ce qu'**elle comprend les étapes de :
a) soumettre des cellules infectées par un pathogène bactérien, protozoaire ou parasitaire intracellulaire à un stress par chaleur ou facteur onconécrosant ; et
b) extraire des complexes de fragments peptidiques antigéniques / protéines de choc endogènes des cellules stressées ; et
c) utiliser les produits extraits comme déterminant immunogène dans la préparation de la composition de vaccin.

2. Une méthode telle que revendiquée dans la revendication 1, **caractérisée en ce que** le principe actif du déterminant immunogène consiste principalement en un ou plusieurs complexes de fragments peptidiques antigéniques / protéines de choc.

3. Une méthode telle que revendiquée dans l'une ou l'autre des revendications 1 et 2, **caractérisée en ce que** les cellules sont infectées par des pathogènes bactériens et le stress appliqué est la chaleur.

4. Une méthode telle que revendiquée dans la revendication 3,
**caractérisée en ce que** le stress dû à la chaleur est obtenu par chauffage à de 5 à 8° au-dessus de la température de culture normale des cellules.

5. Une méthode telle que revendiquée dans la revendication 1, **caractérisée en ce que** les cellules sont infectées par des pathogènes parasitaires et le stress est induit par facteur onconécrosant.

6. Une méthode telle que revendiquée dans l'une quelconque des revendications précédentes, **caractérisée en ce que** les cellules ont été modifiées pour induire la synthèse de protéines de stress.

7. Une composition de vaccin contenant un déterminant immunogène, **caractérisée en ce que** le déterminant immunogène est produit par une méthode telle que revendiquée dans l'une quelconque des revendications 1 à 6.

8. Une composition de vaccin telle que revendiquée dans la revendication 7, **caractérisée en ce que** la composition contient également un adjuvant pour le déterminant immunogène.

9. Une composition de vaccin telle que revendiquée dans les revendications 7 ou 8, **caractérisée en ce que** la composition est une composition aqueuse.

10. Utilisation d'une composition de vaccin telle que revendiquée dans l'une quelconque des revendications 7 à 9 dans la préparation d'un médicament destiné à déclencher une réponse immunitaire chez un animal.

11. L'utilisation telle que revendiquée dans la revendication 10, **caractérisée en ce que** la composition de vaccin est destinée à être administrée par injection.
